Europäisches Patentamt

**European Patent Office**

**Office européen des brevets**

(19)

(11) Numéro de publication: **0 151 892**
**A1**

(12)

# DEMANDE DE BREVET EUROPEEN

(21) Numéro de dépôt: **84402310.1**

(22) Date de dépôt: **14.11.84**

(51) Int. Cl.⁴: **A 61 B 17/56**

(30) Priorité: **15.11.83 FR 8318141**

(43) Date de publication de la demande:
**21.08.85 Bulletin 85/34**

(84) Etats contractants désignés:
**AT BE CH DE FR GB IT LI LU NL SE**

(71) Demandeur: **SANORTHO, Société anonyme dite**
**4 rue Quentin Bauchart**
**F-75008 Paris(FR)**

(72) Inventeur: **Mathieu, Michel**
**15 Square Eblé**
**F-78150 Le Chesnay(FR)**

(74) Mandataire: **Combe, André**
**CABINET BEAU DE LOMENIE 55 rue d'Amsterdam**
**F-75008 Paris(FR)**

(54) **Dispositif pour la manipulation d'ostéosynthèses et plus particulièrement de pièces dites "clou - plaque" et "lame - plaque".**

(57) La présente invention concerne une clef permettant la manipulation de pièces d'ostéosynthèses dites clou-plaque et lameplaque, caractérisée en ce qu'elle est constituée de deux parties cylindriques dont la première (2) est terminée par une partie (3) de section non circulaire et dont la seconde (5) est terminée par une tête (6), ladite clef étant creuse et contenant une tige (7) dont une extrémité filetée (8) sort de quelques millimètres audelà de l'extrémité de la partie (3), ladite tige pouvant être manipulée en rotation grâce à une molette (9) goupillée sur ladite tige et sortant d'une fenêtre ménagée dans ladite seconde partie cylindrique (5).

Fig.1

EP 0 151 892 A1

La présente invention concerne un dispositif pour la manipulation, notamment la mise en place d'ostéosynthèses et plus particulièrement de pièces dites "clou-plaque" et "lame-plaque".

Dans le brevet français 1 443 965, on a décrit un dispositif pour la manipulation, notamment pour la mise en place, d'un "clou-plaque". Ce dispositif se compose d'une clef spéciale et d'un porte clou. Ladite clef-spéciale comporte une portion héxagonale centrale de part et d'autre de laquelle se situent deux parties cylindriques. A l'extrémité libre de l'une des parties cylindriques, on a ménagé trois ergots qui sont destinés à s'enfoncer dans trois trous convenablement positionnés dans le "clou-plaque". Sur toute sa longueur la clef spéciale est pourvue d'un forage axial. Le porte clou, destiné à passer à l'intérieur du forage axial de ladite clef spéciale, est essentiellement pourvu , à l'une de ses extrémités d'une partie filetée de diamètre et de pas correspondant à un filetage ménagé  dans le clou-plaque et à son autre extrémité d'une tête. La manipulation du "clou-plaque", à l'aide dudit dispositif nécessite l'emploi d'un marteau spécial décrit en lui-même et par son utilisation dans ce même brevet français.

Or, ledit dispositif présente certaines difficultés d'utilisation, notamment en ce qui concerne l'usage des ergots et une relative fragilité du "porte-clou". La présente invention vise à surmonter ces difficultés.

Le dispositif selon la présente invention est une clef en métal moulé ou forgé, formée d'un corps constitué de deux parties cylindriques, dont l'une, par exemple de section circulaire, est terminée par une partie de section non circulaire et dont l'autre, dont la section est d'une forme permettant la préhension, est terminée par une tête, ladite clef étant creuse et contenant une tige dont une extrémité filetée sort de quelques millimètres à l'extrémité de la section non circulaire de ladite première partie cylindrique, ladite tige pouvant être manipulée en rotation grâce à une molette goupillée sur ladite tige, sortant d'une fenêtre ménagée dans ladite deuxième partie cylindrique.

Bien évidemment, la clef selon l'invention sera vissée, grâce à l'extrémité filetée de sa tige, dans un trou convenable fileté de la pièce "clou-plaque" ou "lame-plaque" et la partie non circulaire de ladite première partie cylindrique de ladite clef, viendra, lors de ce vissage, s'encastrer dans un logement de forme correspondante aménagé autour dudit trou de la pièce "clou-plaque" ou "lame-plaque".

L'invention sera mieux comprise dans ses diverses possibilités de réalisation en se référant aux figures 1, 2, 3 et 4.

La figure 1 est une vue en élévation d'une clef selon l'invention.

La figure 2 est une vue en bout (ou vue de gauche) de ladite clef.

La figure 3 est une vue, en élévation, d'un "clou-plaque" que la clef selon l'invention, permet de manipuler.

La figure 4 est une vue en bout, (ou vue de droite) dudit "clou-plaque".

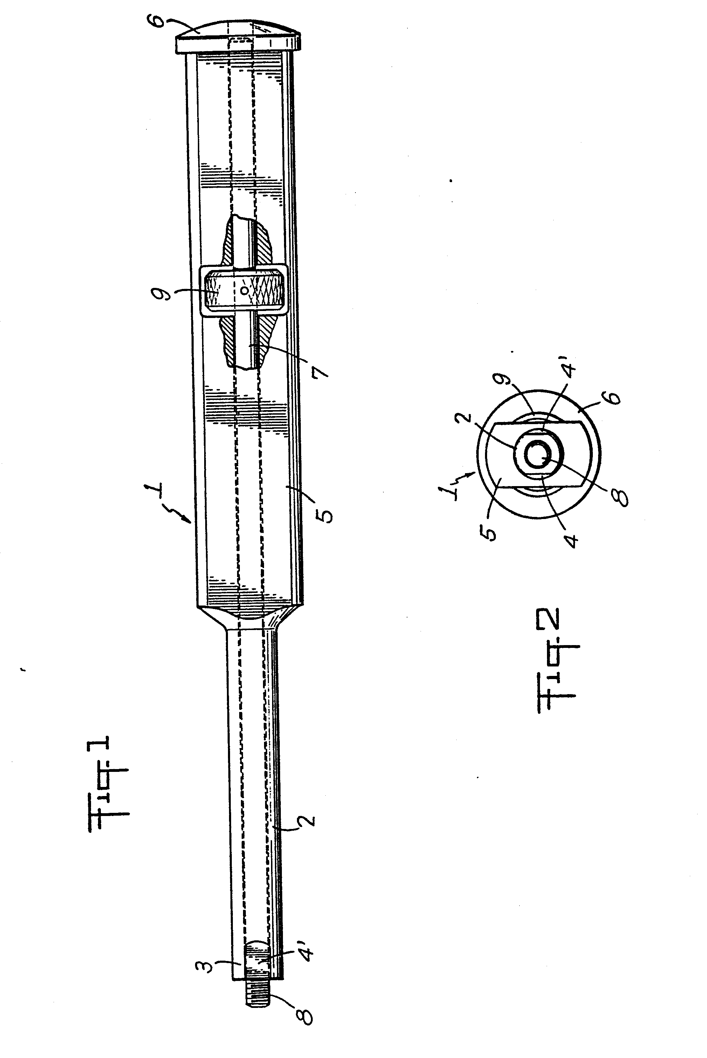

Sur la figure 1, on voit en 1 le corps de la clef. Ce corps est constitué par :

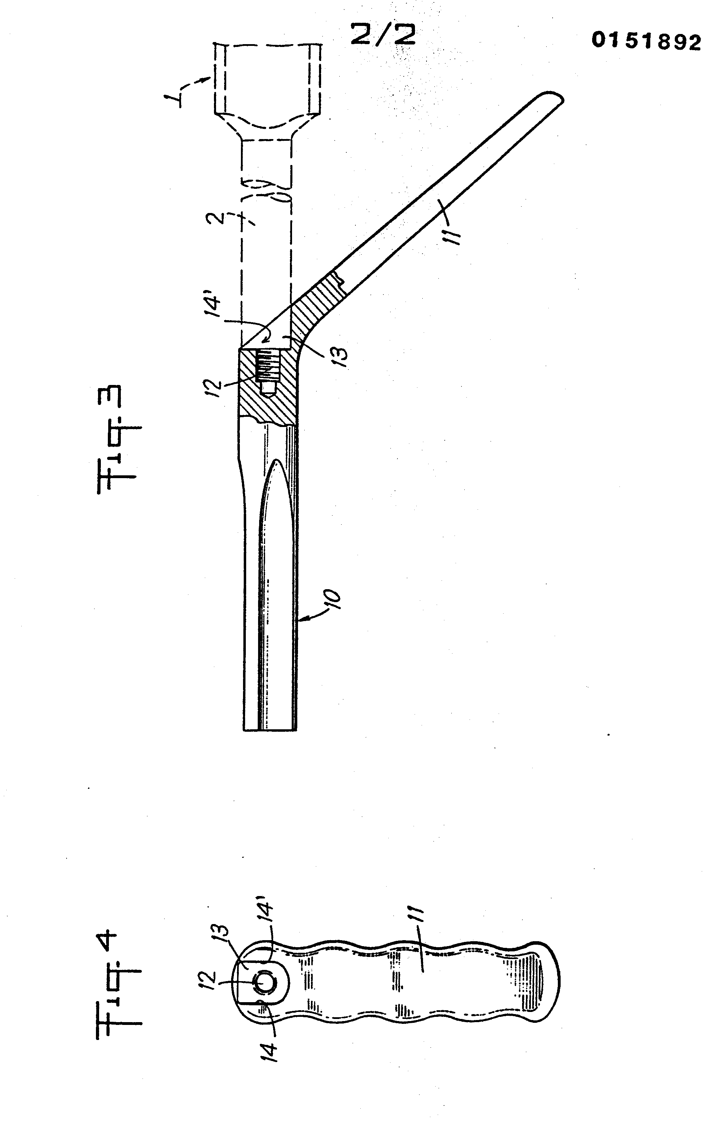

- 2 une partie cylindrique de section par exemple circulaire et de diamètre relativement faible (par exemple compris entre 0,8 et 1,5 cm). Cette extrémité cylindrique à section circulaire est terminée par une partie 3 présentant une section non circulaire; sur l'exemple des figures 1 et 2, on a choisi de réaliser cette section non circulaire de la partie 3 en ménageant deux méplats 4,4' (fig. 2)

- 5 une partie cylindrique de forme permettant la préhension sur l'utilisation ; dans l'exemple choisi, la section droite de cette partie 5 a sensiblement (cf fig. 2) une forme rectangulaire constituée de deux plans parallèles réunis par deux parties légèrement bombées. A l'extrémité de cette partie cylindrique 5, on a ménagé une tête 6.

A l'intérieur du corps de la clef, on a ménagé un forage axial dans lequel, on a introduit une tige 7 qui pivote librement dans ledit forage ; cette tige 7 comporte une extrémité filetée 8 qui sort de quelques millimètres au delà de l'extrémité 3 de la partie cylindrique 2 de la clef ; sur cette tige, on a goupillé une molette 9 qui, dépassant au-delà des plans parallèles qui forment les côtés de la partie cylindrique 5, permet d'entraîner ladite tige en rotation. Cette tige 7 est perforée selon son axe, sur

au moins une partie de sa longueur, de façon à laisser passer une broche. Pour comprendre l'utilisation de la clef selon l'invention, on a représenté schématiquement un clou-plaque sur les figures 3 et 4 ; ce clou-plaque est constitué d'un corps 10 et d'une plaque 11 ; le corps 10 a, dans sa partie voisine de la plaque, une forme cylindrique et présente sur le reste de sa longueur des gorges plus ou moins profondes ; l'axe du corps 10 est incliné d'un angle donné par rapport à la direction de la plaque 11.

Dans la partie du corps qui raccorde avec la plaque, on a ménagé un trou fileté 12 ; le diamètre et le pas de vis de ce trou correspondent au diamètre et au pas de vis de l'extrémité filetée 8 de la tige 7. Autour de ce trou, on a aménagé un logement 13 dont la forme et les dimensions sont telles, qu'elles s'adaptent exactement aux formes et dimensions de la partie 3, de section non cylindrique, de la clef ; ainsi dans l'exemple particulier représenté, la forme non circulaire de la partie 3 ayant été obtenue par deux méplats 4 et 4', le logement 13 comportera deux méplats 14 et 14' sur lesquels les méplats 4 et 4' viendront glisser lorsque la clef sera vissée, par sa partie filetée 8, dans le trou 12 du "clou-plaque".

On se rend compte que la forme de la section droite de la partie cylindrique 2 a peu d'importance. Par contre, la forme de la section droite de l'extrémité 3 de cette partie cylindrique doit être telle que, coopérant avec la forme du logement ménagé dans le clou-plaque, elle empêche toute rotation de la clef lorsque celle-ci est vissée sur ledit "clou-plaque" ; en fait, la forme de cette section droite de l'extrémité 3 ne doit pas être circulaire mais peut-être triangulaire, hexagonale, elliptique ou comporter deux méplats comme dans l'exemple choisi.

La forme de la section droite de la partie cylindrique 5 est de peu d'importance ; il convient cependant de faire en sorte, que ladite partie puisse être appréhendée facilement et solidement par un opérateur et qu'elle permette l'utilisation de marteaux spéciaux aptes non seulement à frapper sur la face externe de la tête pour enfoncer le clou, mais également à frapper sur la face interne de ladite tête pour sortir ledit clou.

La clef selon l'invention, et les "clou-plaque" et "lame-plaque" avec lesquels ladite clef est utilisable - et qui ont donc reçu les aménagements (filetage convenable et logement de forme adaptée) nécessaires pour que l'utilisation de cette clef soit possible - présentent pour les opérateurs des avantages substantiels par rapport aux dispositifs antérieurement connus. Ces avantages sont essentiellement, une plus grande facilité d'emploi, dont une plus grande fiabilité, et une solidité améliorée.

## R E V E N D I C A T I O N S

1.      Clef permettant la manipulation de pièces d'ostéosynthèses dites clou-plaque et lame-plaque caractérisée en ce qu'elle est
constituée de deux parties cylindriques dont la première (2) est
terminée par une partie (3) de section non circulaire et dont la
seconde (5) est terminée par une tête (6) ladite clef étant creuse
et contenant une tige (7) dont une extrémité filetée (8) sort de
quelques millimètres au-delà de l'extrémité de la partie (3), ladite tige pouvant être manipulée en rotation grâce à une molette
(9) goupillée sur ladite tige et sortant d'une fenêtre ménagée dans
ladite seconde partie cylindrique (5).

2.      Pièces d'ostéosynthèses dites "clou-plaque" et "lame-
plaque" dont la manipulation est réalisée avec une clef selon la
revendication 1 caractérisée en ce que l'extrémité du corps (10)
se raccordant à la plaque 11 comporte un trou fileté pouvant coopérer avec l'extrémité filetée(8) de la tige contenue dans la clef
et un logement (13) coopérant avec la partie (3) de section non
circulaire de la clef.

Fig.1

Fig.2

0151892

Fig.3

Fig.4

Office européen
des brevets

**RAPPORT DE RECHERCHE EUROPEENNE**

**0151892**

Numéro de la demande

EP 84 40 2310

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| D,Y | FR-A-1 443 965 (DESCAMPS) <br> * Page 5, colonne de gauche, alinéas 3-7; figures 47-50 * | 1 | A 61 B 17/18 |
| X | * Page 4, colonne de droite, avant dernier alinéa; page 5, colonne de gauche, alinéa 3; figures 43-46 * | 2 | |
| Y | --- <br> DE-A-2 240 336 (FISCHER) <br> * Pages 5,6; figure 1 * | 1 | |
| | ----- | | |

DOMAINES TECHNIQUES RECHERCHES (Int. Cl.4)

A 61 B

Le présent rapport de recherche a été établi pour toutes les revendications

| Lieu de la recherche <br> LA HAYE | Date d'achèvement de la recherche <br> 27-02-1985 | Examinateur <br> LEMERCIER D.L.L. |
|---|---|---|